# EUROPEAN PATENT APPLICATION

(11) **EP 3 241 549 A1**
(43) Date of publication of application: **08.11.2017**
(21) Application number: 17169102.5
(22) Date of filing: 02.05.2017
(51) Int. Cl.: A61K 9/48, A61K 9/50, A61K 31/137

(54) **SPRINKLE COMPOSITION OF CINACALCET**

(30) Priority: 02.05.2016 IN 201611015199
(71) Applicant: Sun Pharmaceutical Industries Limited, 400 063 Mumbai MAH (IN)
(72) Inventor: YADAV, Avaneesh, 205135 Firozabad, Uttar Pradesh (IN); DHALIWAL, Mona, 247001 Saharanpur, Uttar Pradesh (IN); GARG, Mukesh Kumar, 122018 Gurgaon, Haryana (IN)
(74) Representative: Cronin, Brian Harold John

(57) **Abstract**

The present invention relates to a sprinkle composition of cinacalcet which provides a dissolution profile which is comparable to the currently marketed tablet dosage form.

## Description

### Field of the Invention

The present invention relates to a sprinkle composition of cinacalcet which provides a dissolution profile which is comparable to the currently marketed tablet dosage form. The sprinkle composition of the present invention is in the form of capsule dosage form comprising coated cores.

### Background of the Invention

Cinacalcet is a calcium-sensing receptor agonist. It is commercially available as tablets (Sensipar^{®}) in US since March 2004 and is indicated for treatment of secondary hyperparathyroidism resulting from chronical kidney disease and for the treatment of hypercalcemia in patients with either parathyroid carcinoma or hyperparathyroidism. Currently, cinacalcet is also in phase 3 clinical trials in pediatric patients with secondary hyperparathyroidism (SHPT) and chronic kidney disease (CKD) on dialysis wherein cinacalcet capsules are sprinkled onto soft food or suspended into a liquid suspension for oral administration.

PCT Publication No. WO 2012/071535 discloses a hard shell capsule containing a granular powder formulation of cinacalcet which can be sprinkled on and mixed with food or drinks and then administered orally to the pediatric patients.

Cinacalcet is known to possess a bitter taste as well asinduces numbness of the tongue, hence the granular compositions disclosed in the above PCT application may not able to provide desired taste mask or minimize the feeling of numbness.

Hence, taste masked sprinkle compositions of cinacalcet comprising coated cores remain highly desirable so as to provide enhanced palatability and improved patient compliance. On the other hand, coating is known to hamper the release of the drug from the core, and this is more critical in an immediate release composition.

Thus, there exists a need in the art to formulate a sprinkle composition of cinacalcet which ispalatable and provides dissolution profile which is comparable to the currently marketed tablet dosage form.

### Summary of the Invention

The present invention relates in part to a sprinkle composition of cinacalcet comprising a coated core of cinacalcet wherein at least about 75% of cinacalcet is released from the composition in 60 minutes in 900 mL of 0.05N hydrochloric acid in USP II apparatus at 100 rpm.

### Detailed Description of the Invention

A first aspect of the present invention provides a capsule composition of cinacalcet comprising a coated core of cinacalcet wherein at least about 60 % of cinacalcet is released from the composition in 60 minutes in 900 mL of 0.05N hydrochloric acid in USP II apparatus at 100 rpm.

According to one of the embodiments of this aspect, the capsule composition releases at least about 40 % of cinacalcet from the composition in 30 minutes in 900 mL of 0.05N hydrochloric acid in USP II apparatus at 100 rpm.

According to another embodiment of this aspect, the composition comprises one or more coated core of cinacalcet.

According to another embodiment of this aspect, the coated core comprises cinacalcet granules or beads coated with a taste masked coating. The cinacalcet granules or beads in the coated core comprise sweeteners.

According to another embodiment of the above aspect, taste mask coating comprises taste mask polymer.

According to yet another embodiment of the above aspect, taste mask coating comprises sweeteners.

According to another aspect embodiment of the above aspect, the core comprises an inert core coated with a coating comprising cinacalcet. The weight ratio of cinacalcet to inert core is from about 1:0.1 to about 1:4. This core is further coated with a taste masked coating composition to form a coated cores.

According to one embodiment of this aspect, the taste masked coating is from about 2% to about 40% w/w of the total weight of the composition.

The coated cores have a particle size d₉₀ of less than or equal to 800µm.

The capsule composition further comprises one or more pharmaceutically acceptable excipients selected form the group comprising binder, disintegrant, diluents, surfactant, sweetener, lubricants, glidants, coloring agents, flavoring agents, and mixtures thereof.

For example, in an embodiment, provided herein is a pharmaceutically acceptable capsule containing a drug coated core composition comprising: a plurality of coated cores and a lubricant, wherein each coated core comprises a) an inert core,b) a drug layer comprising cinacalcet or pharmaceutically acceptable salt thereof, wherein the drug layer covers the inert core, and c) a taste mask layer (e.g., that may comprise hypromellose) covering the drug layer; and wherein the capsule and/or the plurality of coated cores releases at least about 60 % of cinacalcet in 60 minutes when placed in 900 mL of 0.05 N hydrochloric acid in USP II apparatus at 100 rpm; and/orcapsule or plurality of coated cores releases at least about 40 % of cinacalcet in 30 minutes when placed in 900 mL of 0.05 N hydrochloric acid in USP II apparatus at 100 rpm.

In some embodiments, the drug layer comprises cinacalcet hydrochloride where the weight ratio of cinacalcet hydrochloride to inert core is from about 1.25:1 to about 2:1, wherein in some embodiments, the drug layer further comprises hypromellose.

According to another embodiment of this aspect, the ratio of lubricant: disintegrant: glidant is from about 0.5:0.5:1.0 to 2:2:2.5. For example, in some embodiments, this ratio of the pharmaceutically acceptable excipients may be critical for the disintegration of the capsule in the dissolution medium. For example, disclosed pharmaceutically acceptable capsules may further comprise a disintegrant (e.g., croscarmellose sodium and glidant (e.g., magnesium stearate), wherein the ratio of lubricant: disintegrant: glidant (e.g., silicon dioxide) is from about 0.5:0.5:1.0 to 2:2:2.5

According to one embodiment of this aspect, the disintegrant is present intragranularly as well as extragranularly. The disintegrant can be added, for example, at the lubrication stage.

A second aspect of the present invention provides a process for the preparation of a capsule composition of cinacalcet comprising:
(i) dispersing or dissolving cinacalcet with one or more pharmaceutically acceptable excipients in a suitable solvent to obtain a dispersion or a solution;
(ii) coating an inert core with the dispersion or solution of step (i) to obtain the drug coated core;
(iii) applying a taste mask coating on the drug coated core of step (ii); and
(iv) blending the coreof step (iii) with one or more pharmaceutically acceptable excipients and filling into suitable sized capsules.

A third aspect of the present invention provides a process for the preparation of a capsule composition of cinacalcet comprising:
(i) preparing cinacalcet granules using a wet or a dry granulation technique;
(ii) coating the cinacalcet granules with taste mask coating to obtain a coated core;
(iii) blending the coated core of step (ii) with one or more pharmaceutically acceptable excipients and filling into suitable sized capsules.

The term "cinacalcet" refers to cinacalcet base as well as its hydrochloride salt. It may be present in the sprinkle composition in an amount from about 5 mg to about 100mg. Cinacalcet is present in the sprinkle composition in an amount from about 10% to about 70% by weight based on the total weight of the composition.

The term "sprinkle composition" as used herein refers to a composition which can be sprinkled on to the soft food such as apple sauce, yoghurt, pudding or drinks, and then administered orally to the patients. The composition may also be administered through NG tube in patients who have difficulty in swallowing.

The inert core may be an inert non-pareil sugar spheres, microcrystalline cellulose spheres, or glass beads.

Examples of fillers or diluents include, but not limited to, lactose, sorbitol, calcium dihydrogen phosphate dihydrate, calcium phosphate-dibasic, calcium phosphate-tribasic, calcium sulfate, microcrystalline cellulose, silicified microcrystalline cellulose, mannitol, starch, pregelatinized starch, and mixtures thereof. These may be present in the composition in the range from about 30% to about 95% of the total weight of the composition.

Examples of binders include, but not limited to, corn starch, pregelatinized starch, microcrystalline cellulose, silicified microcrystalline cellulose, methyl cellulose, hydroxypropyl cellulose (HPC-L), methylcellulose, carboxymethyl cellulose sodium, hydroxypropyl methylcellulose, polyvinylpyrrolidone, and mixtures thereof. These may be present in the composition in the range from about 5% to about 60% of the total weight of the composition.

Examples of disintegrants include, but not limited to, cross-linked polyvinyl pyrrolidone, corn starch, and modified starches, agar-agar, calcium carbonate, sodium carbonate, alginic acids, croscarmellose sodium, sodium starch glycolate, microcrystalline cellulose, hydroxypropyl cellulose (L-HPC), and mixtures thereof. These may be present intragranularly or extragranularly. The disintegrant may be present in the composition in the range from about 0.1% to about 15% of the total weight of the composition.

Examples of lubricants and glidants include, but not limited to, colloidal anhydrous silica, stearic acid, magnesium stearate, glyceryl behenate, calcium stearate, sodium stearyl fumarate, stearic acid, talc, microcrystalline wax, yellow beeswax, white beeswax, and mixtures thereof. These may be present in the composition in the range from about 0.01 % to about 10% of the total weight of the composition.

Examples of surfactants include, but not limited to, sorbitanmonostearate, polyoxythylenesorbitanmonostearate, *e.g.*,Polysorbate 60 or Polysorbate 80, non-ethoxylated glyceryl monostearate, cetomacrogol, cetostearyl alcohol, sodium stearoyllactylate, lecithin, and mixtures thereof. These may be present in the composition in the range from about 0.1% w/w to about 20% w/w of the total weight of the composition.

Examples of sweeteners include, but not limited to, sucrose, sucralose, sorbitol, xylitol, dextrose, fructose, maltitol, acesulfame potassium, aspartame, saccharin, saccharin sodium, maltitol, glucose, cyclamate, sodium cyclamate, and mixtures thereof. These may be present in the composition in the range from about 0.1% w/w to about 20% w/w of the total weight of the composition.

The coloring agents and flavoring agents of the present invention may be selected from any FDA approved colors or flavors for oral use.

The taste mask coating may comprise one or more taste mask polymers and coating additives. Pharmaceutically acceptable coating additives may be sweeteners, pore formers, plasticizers, anti-tacking agent, opacifiers, coloring agents, coating agent, and mixtures thereof.

Suitable taste mask polymers are selected from the group comprising water soluble polymers such as hydroxyl ethyl cellulose, hydroxyl propyl cellulose, hypromellose, or water insoluble polymers such as ethyl cellulose, polycarbophil, polyacrylic acid, and mixtures thereof. Taste mask polymers may also be enteric such as cellulose acetate butyrate, cellulose acetate phthalate, ethyl vinyl phthalate, polyvinyl acetate phthalate, hydroxy alkyl cellulose phthalates, methacrylic acid/ethyl acrylate copolymers or mixtures thereof. In particular, the taste mask polymer is ethyl cellulose. These may be present in the composition in the range from about 0.01% w/w to about 20% w/w of the composition.

Examples of pore formers include calcium carbonate, calcium phosphate, calcium saccharide, calcium succinate, calcium tartrate, ferric acetate, ferric hydroxide, ferric phosphate, magnesium carbonate, magnesium citrate, magnesium hydroxide, magnesium phosphate, hypromellose, e.g. HPMC E5, and mixtures thereof. These may be present in the composition in the range from about 0.01% w/w to about 20% w/w of the composition.

Examples of plasticizers include propylene glycol, triethyl citrate, tributyl citrate, dibutyl sebacate, acetyl tributyl citrate, glyceryl monostearate, triacetin, polyethylene glycol, diethyl phthalate, acetylated monoglycerides, diacetylated monoglycerides, cetyl alcohol, and mixtures thereof. These may be present in the composition in the range from about 0.01% w/w to about 15% w/w of the composition.

Examples of anti-tacking agent include talc, glyceryl monostearate, vegetable oil, waxes, a blend of magnesium stearate and sodium lauryl sulfate, boric acid, sodium benzoate, sodium acetate; sodium chloride, polyethylene glycol, sodium oleate, sodium lauryl sulfate, magnesium lauryl sulfate, corn starch, amorphous silicon dioxide, Vitamin E, Vitamin E TPGS, and mixtures thereof. These may be present in the composition in the range from about 0.01% w/w to about 15% w/w of the composition.

Examples of opacifiers include titanium dioxide, manganese dioxide, iron oxide, silicon dioxide, and mixtures thereof. These may be present in the composition in the range from about 0.01% w/w to about 15% w/w of the composition.

Suitable solvents are selected from the group comprising purified water, ethyl alcohol, isopropyl alcohol, acetone, and mixtures thereof. These may be present in the composition in the range from about 0.01 % w/w to about 20% w/w of the composition.

The coating may be carried out by using any conventional coating techniques known in the art, such as spray coating using fluidized bed processor or pan coating.

The term "about" as used herein, refers to any value which lies within the range defined by a variation of up to ±10% of the value.

The following examples represent various embodiments according to the present invention.

The examples are given solely for the purpose of illustration and are not to be construed as limitations of the present invention, as many variations thereof are possible without departing from the spirit and scope of the invention.

### Example 1:

| **Ingredients** | **mg/capsule** |
|---|---|
| **Drug coated cores** | |
| Cinacalcethydrochloride | 99.18 |
| Sugar sphere | 50.00 |
| Hypromellose (HPMC E5) | 29.75 |
| Polysorbate 80 | 2.00 |
| Sucralose | 4.00 |
| Purified water | q.s. |

| **Taste mask coating** | |
|---|---|
| Hypromellose (HPMC E5) | 31.77 |
| Ethyl cellulose | 7.95 |
| Sucralose | 7.20 |
| Talc | 8.57 |
| Isopropyl Alcohol | q.s. |
| Water | q.s. |

| **Lubrication** | |
|---|---|
| Magnesium stearate | 2.40 |
| Colloidal silicon dioxide | 3.61 |
| Croscarmellose sodium | 2.40 |

### Manufacturing Process:

1. Cinacalcet hydrochloride, hypromellose, polysorbate 80 and sucralose were dispersed in purified water to obtain a drug dispersion.
2. Sugar spheres were coated with the drug dispersion of step 1 to obtain drug coated core.
3. Hypromellose, ethyl cellulose, sucralose and talc were dispersed in a mixture of isopropyl alcohol and purified water to obtain a dispersion.
4. The drug coated cores of step 2 were coated with the dispersion of step 3 to obtain coated cores.
5. Magnesium stearate, croscarmellose sodium and colloidal silicon dioxide were mixed with coated coresof step 4 to obtain a lubricated beads.
6. The lubricated beads of step 5 was filled into suitable sized capsules.

### Example 2:

| **Ingredients** | **mg/capsule** |
|---|---|
| **Drug coated cores** | |
| Cinacalcethydrochloride | 99.18 |
| Sugar sphere | 75.00 |
| Hypromellose (HPMC E5) | 29.75 |
| Polysorbate 80 | 2.00 |
| Sucralose | 4.00 |
| Purified water | q.s. |

| **Taste mask coating-part-1** | |
|---|---|
| Hypromellose (HPMC E5) | 24.05 |
| Ethyl cellulose | 6.01 |
| Sucralose | 5.45 |
| Talc | 6.48 |
| Isopropyl Alcohol | q.s. |
| Water | q.s. |

| **Taste mask coating-part-2** | |
|---|---|
| Hypromellose (HPMC E5) | 4.06 |
| Ethyl cellulose | 9.47 |
| Sucralose | 1.88 |
| Magnesium stearate | 0.57 |
| Talc | 2.92 |
| Isopropyl alcohol | q.s. |
| Water | q.s. |

| **Lubrication** | |
|---|---|
| Magnesium stearate | 2.71 |
| Colloidal silicon dioxide | 4.07 |
| Croscarmellose sodium | 2.71 |

### Manufacturing Process:

1. Cinacalcet hydrochloride, hypromellose, polysorbate 80 and sucralose were dispersed in purified water to obtain a drug dispersion.
2. Sugar spheres were coated with the drug dispersion of step 1 to obtain drug coated core.
3. Hypromellose, ethyl cellulose, talc and sucralose were dispersed in a mixture of isopropyl alcohol and purified water to obtain a dispersion.
4. The drug coated cores of step 2 were coated with the dispersion of step 3.
5. Hypromellose, ethyl cellulose, magnesium stearate, sucralose and talc were dispersed in a mixture of isopropyl alcohol and purified water to obtain a dispersion.
6. The coated cores of step 4 were further coated with the dispersion of step 5.
7. Magnesium stearate, croscarmellose sodium and colloidal silicon dioxide were mixed with coated coresof step 6 to obtain lubricated beads.
8. The lubricated beads of step 7 were filled into suitable sized capsules.

### Dissolution Study

Table 1 provides a comparison of the dissolution profile of pharmaceutical compositions of Examples 1 and 2 vis-à-vis marketed cinacalcet tablets (Sensipar^{®}). The dissolution was performed in 900 mL of 0.05 N hydrochloric acid in USP II apparatus at 100 rpm for 90 minutes, with sinkers. The samples were analyzed by high performance liquid chromatography (HPLC)/ UV. As seen from the results, the pharmaceutical compositions (Examples 1 and 2) and Sensipar^{®} tablets achieved similar dissolution profile.

**Table 1: Percentage of cinacalcet released in the dissolution media(0.05 N hydrochloric acid)**

| **Pharmaceutical Composition** | **Time point (in minutes)** | | | | | |
|---|---|---|---|---|---|---|
| | 10 | 15 | 30 | 45 | 60 | 90 |
| **Marketed tablet (Sensipar^{®})** | 73 | 84 | 92 | 96 | 97 | 98 |
| **Example 1** | 66 | 87 | 96 | 98 | 99 | 99 |
| **Example 2** | 12 | 54 | 75 | 87 | 91 | 97 |

## Claims

1. A capsule composition comprising a coated core of cinacalcet wherein the said composition releases at least about 60 % of cinacalcet in 60 minutes in 900 mL of 0.05 N hydrochloric acid in USP II apparatus at 100 rpm and said coated cores are taste masked.

2. The capsule composition according to claim 1 wherein the composition further releases at least about 40 % of cinacalcet in 30 minutes in 900 mL of 0.05 N hydrochloric acid in USP II apparatus at 100 rpm.

3. The capsule composition according to claim 1 wherein the coated cores are selected from the group consisting of plurality of granules, beads, pellets and minitablets.

4. The capsule composition according to claim 3 wherein the coated cores have the particle size d₉₀ of less than or equal to 800 µm.

5. The capsule composition according to claim 1 wherein the coated core comprises
a) an inert core,
b) a drug layer comprising cinacalcet coated over the inert core, and
c) a taste mask coating over the cores of b) to form a coated core.

6. The capsule composition according to claim 5 wherein the weight ratio of cinacalcet to inert core is from about 1:0.1 to about 1:4.

7. The capsule composition according to claim 5 wherein the taste mask coating may comprise one or more sweetener, taste mask polymers and coating additives.

8. The capsule composition according to claim 5 wherein the taste mask coating is present in an amount from about 2% to about 40% w/w of the total weight of the composition.

9. The capsule composition according to claim 5, wherein the composition further comprises one or more pharmaceutically acceptable excipients selected form the group comprising binder, disintegrant, diluents, surfactant, sweetener, antioxidants, glidants, and lubricants.

10. The capsule composition according to claim 9, wherein the binder is present in an amount from about 8% to about 50% of the total weight of the composition.

11. The capsule composition according to claim 9, wherein the disintegrant is present extra granularly.

12. A capsule composition of cinacalcet comprising coated cores, wherein the coated cores comprise:
a) an inner core comprising cinacalcet, and
b) a taste mask coating over the inner core
wherein the coated cores are capable of sprinkling on soft food.

13. A capsule composition of cinacalcet comprising coated cores in the form of
a) an inert core,
b) a drug layer comprising cinacalcet coated over the inert core, and
c) a taste mask coating over the cores of b) to form coated cores
wherein the weight ratio of cinacalcet to inert core is from about 1:0.1 to about 1:4.

14. The capsule composition according to claim 13, wherein the coated cores can be sprinkled on soft food.

15. A pharmaceutically acceptable capsule containing a drug coated core composition comprising: a plurality of coated cores and a lubricant, wherein each coated core comprises
a) an inert core,
b) a drug layer comprising cinacalcet or pharmaceutically acceptable salt thereof, wherein the drug layer covers the inert core, and
c) a taste mask layer covering the drug layer;
wherein the capsule releases at least about 60 % of cinacalcet in 60 minutes when placed in 900 mL of 0.05 N hydrochloric acid in USP II apparatus at 100 rpm.

16. The pharmaceutically acceptable capsule of claim 15, wherein the capsule releases at least about 40 % of cinacalcet in 30 minutes when placed in 900 mL of 0.05 N hydrochloric acid in USP II apparatus at 100 rpm.

17. The pharmaceutically acceptable capsule according to claim 15 wherein the drug layer comprises cinacalcet hydrochloride and the weight ratio of cinacalcet hydrochloride to inert core is from about 1.25:1 to about 2:1.

18. The pharmaceutically acceptable capsule of claim 15, wherein the taste mask layer comprises hypromellose and a sweetener.

19. The pharmaceutically acceptable capsule of claim 15, wherein the drug layer further comprises hypromellose.

20. The pharmaceutically acceptable capsule of claim 15, further comprising a disintegrant and glidant, wherein the ratio of lubricant: disintegrant: glidant is from about 0.5:0.5:1.0 to 2:2:2.5.

21. The pharmaceutically acceptable capsult of claim 20, wherein the disintegrant is croscarmellose sodium, the lubricant is magnesium stearate, and the glidant is silicon dioxide.
